# EUROPÄISCHE PATENTSCHRIFT

(11) **EP 1 253 885 B1**
(45) Veröffentlichungstag und Bekanntmachung des Hinweises auf die Patenterteilung: **26.07.2006**
(21) Anmeldenummer: 01921274.5
(22) Anmeldetag: 08.02.2001
(51) Int. Cl.: A61F 5/01

(54) **ORTHESE MIT VERSTELLUNG DES FLEXIONS- UND EXTENSIONSANSCHLAGS DURCH SCHIENENSCHWENKBEWEGUNGEN**
ORTHESIS COMPRISING A FLEXION AND AN EXTENSION STOP THAT CAN BE ADJUSTED BY MEANS OF RAIL PIVOTING MOVEMENTS
ORTHESE AVEC DEPLACEMENT DE LA BUTEE DE FLEXION ET DE LA BUTEE D'EXTENSION PAR MOUVEMENTS DE PIVOTEMENT D'UN RAIL

(30) Priorität: 10.02.2000 DE 10005764
(43) Veröffentlichungstag der Anmeldung: 06.11.2002
(73) Patentinhaber: Albrecht GmbH, 83115 Neubeuern (DE)
(72) Erfinder: ALBRECHT, Erich, 83115 Neubeuern (DE); OPAHLE, Hans-Georg, 83024 Rosenheim (DE)
(74) Vertreter: Bauer, Friedrich
(86) Internationale Anmeldenummer: PCT/EP2001/001364
(87) Internationale Veröffentlichungsnummer: WO 2001/058392

(56) Entgegenhaltungen:
- EP-A- 0 956 837
- DE-A- 19 904 554
- US-A- 5 052 379
- US-A- 5 885 235
- US-A- 5 954 677

## Beschreibung

Die Erfindung betrifft eine Orthese zur Reduktion von Streck- und/oder Beugedefiziten im Bereich eines Gelenks, insbesondere eine Ellbogenschiene, gemäß dem Oberbegriff des Anspruchs 1.

Insbesondere Gelenkkapseln und/oder Bindegewebe weisen beispielsweise nach Bänderoperation, Unfällen, Entzündungen etc. häufig ein Streck- und/oder Beugedefizit auf. Dies bedeutet, dass ein distales Körperglied, beispielsweise ein Unterarm, nicht mehr vollständig in seine normale Extensions- oder Flexionslage bezüglich eines proximalen Körperglieds, beispielsweise eines Oberarms, gebracht werden kann.

Um einem derartigen Streck- oder Beugedefizit entgegenzuwirken, wird bekannterweise versucht, die Verkürzungen bzw. Schrumpfungen wieder dadurch zu dehnen, dass das distale Körperglied bezüglich des proximalen Körperglieds mittels einer unter Federvorspannung stehenden Quengelvorrichtung in Form einer Orthese in Flexionsrichtung bis zu einem bestimmten Flexionsanschlag und in Extensionsrichtung bis zu einem bestimmten Extensionsanschlag bewegt wird. Mit zunehmender Mobilisierung des Gelenks beziehungsweise der entsprechenden Sehnen und Bänder werden der Flexions- und Extensionsanschlag entsprechend verstellt.

Bekannte, zum Quengeln verwendete Orthesen, weisen zur Begrenzung des Schwenkbereichs Anschlagstifte auf, die in verschiedene, um die Schwenkachse herum angeordnete Bohrungen eingesteckt werden können. Nachteilig ist hierbei jedoch, dass das Verstellen des Flexions- und Extensionsanschlags nur in groben Schritten, beispielsweise 15 Grad Schritten, möglich ist und die Schwenkbereichsgrenzen daher häufig nicht genau genug eingestellt werden können ( siehe z.B. US 59 54 677 A).

Der Erfindung liegt die Aufgabe zu Grunde, eine Orthese zur Reduktion von Streck- und/oder Beugedefiziten zu schaffen, mit der auf eine einfache, schnelle und genaue Weise der Flexions- und Extensionsanschlag stufenlos einstellbar sind.

Diese Aufgabe wird erfindungsgemäß durch die Merkmale des Anspruchs 1 gelöst. Vorteilhafte Ausführungsformen der Erfindung sind in den weiteren Ansprüchen beschrieben.

Bei der erfindungsgemäßen Orthese ist der Flexionsanschlag auf einer um die Schwenkachse des Schienengelenks drehbaren Flexionsanschlagträgerscheibe und der Extensionsanschlag auf einer um die Schwenkachse des Schienengelenks drehbaren Extensionsanschlagträgerscheibe angeordnet. Die Flexions- und Extensionsanschlagträgerscheibe sind mit einer lösbaren Verriegelungseinrichtung in Wirkverbindung, um ein Drehen der Flexions- und Extensionsanschlagträgerscheibe um die Schwenkachse zu blockieren oder zu ermöglichen. Weiterhin ist eine mit der ersten Schiene drehfest gekoppelte und zusammen mit dieser um die Schwenkachse drehbare Anschlagmitnehmereinrichtung vorgesehen, mit der bei gelöster Verriegelungseinrichtung der Flexionsanschlag durch Schwenken der ersten Schiene bis zur gewünschten Flexionsschwenkbereichsgrenze und der Extensionsanschlag durch Schwenken der ersten Schiene bis zur gewünschten Extensionsschwenkbereichsgrenze bewegbar sind. Durch Verriegeln der Verriegelungseinrichtung sind der Flexionsanschlag an der Flexionsschwenkbereichsgrenze und der Extensionsanschlag an der Extensionsschwenkbereichsgrenze arretierbar.

Für die erfindungsgemäße Orthese ist es somit charakteristisch, dass das Einstellen des Flexionsanschlags an der Flexionsschwenkbereichsgrenze und des Extensionsanschlags an der Extensionsschwenkbereichsgrenze ohne jegliches Werkzeug und ohne Herausziehen und Hineinstecken von irgendwelchen Anschlagstiften möglich ist. Es ist vielmehr möglich, allein durch Schwenken der ersten Schiene bis zur Flexionsschwenkbereichsgrenze den Flexionsanschlag bis zu dieser Grenze zu bewegen, und den Extensionsanschlag durch Verschwenken der ersten Schiene bis zur Extensionsschwenkbereichsgrenze zu bewegen, worauf eine Verriegelungseinrichtung betätigt wird, welche den Flexions- und Extensionsanschlag an den gewählten Schwenkbereichsgrenzen arretiert. Die erste Schiene ist daraufhin gegenüber der zweiten Schiene nur mehr innerhalb der festgelegten Schwenkbereichsgrenzen verschwenkbar. Soll der Flexions-und/oder Extensionsanschlag im Laufe der Behandlung nachgestellt werden müssen, ist es lediglich erforderlich, die Verriegelungseinrichtung zu lösen, worauf durch Verschwenken der ersten Schiene bis zur neuen Schwenkbereichsgrenze der entsprechende Anschlag bis zu dieser Schwenkbereichsgrenze mitgenommen wird und anschließend mittels der Verriegelungseinrichtung wieder festgelegt werden kann.

Die erfindungsgemäße Orthese weist somit eine integrierte Anschlagmitnehmereinrichtung auf, die drehfest mit der ersten Schiene verbunden ist und beim Verschwenken der ersten Schiene den Flexions- und Extensionsanschlag in die gewünschten Positionen bringt.

Gemäß einer vorteilhaften Ausführungsform der Erfindung ist die mit der ersten Schiene drehfest gekoppelte Anschlagmitnehmereinrichtung als Schwenkbereichsbegrenzungsmittel ausgebildet, das an der Flexionsschwenkbereichsgrenze am Flexionsanschlag und an der Extensionsschwenkbereichsgrenze am Extensionsanschlag anschlägt. Dies bedeutet mit anderen Worten, dass die Anschlagmitnehmereinrichtung neben der Funktion, den Flexions- und Extensionsanschlag bei gelöster Verriegelungseinrichtung an die Schwenkbereichsgrenzen zu bewegen, die weitere Funktion hat, bei verriegeltem Flexions- und Extensionsanschlag die erste Schiene daran zu hindern, sich über die eingestellten Schwenkbereichsgrenzen zu bewegen.

Gemäß einer vorteilhaften Ausführungsform besteht die Anschlagmitnehmereinrichtung aus mindestens einer Anschlagmitnehmerscheibe mit einem bogenförmig um die Schwenkachse herumgeführten Längsschlitz, in den der Flexionsanschlag und Extensionsanschlag hineinragen.

Zweckmäßigerweise ist eine an der Flexionsanschlagträgerscheibe und Extensionsanschlagträgerscheibe angreifende Rückführfeder vorgesehen, um den Flexionsanschlag und Extensionsanschlag bei gelöster Verriegelungseinrichtung in ihre Ausgangslagen zurückzuführen.

Gemäß einer vorteilhaften Ausführungsform besteht die Verriegelungseinrichtung aus einer an der zweiten Schiene gehalterten Klemmeinrichtung, welche die Flexionsanschlagträgerscheibe und Extensionsanschlagträgerscheibe übergreift und mittels einer Klemmbetätigungseinrichtung zwischen einer Klemmstellung und einer Freigabestellung bewegbar ist.

Zweckmäßigerweise besteht hierbei die Klemmeinrichtung aus zwei Klemmringen, welche die Flexionsanschlagträgerscheibe und Extensionsanschlagträgerscheibe an ihrer äußeren Umfangsfläche mit engem Spiel übergreifen und in der Klemmstellung ein Drehen der Flexionsanschlagträgerscheibe und Extensionsanschlagträgerscheibe verhindern.

Die Erfindung wird nachfolgend anhand der Zeichnungen beispielshaft näher erläutert. In diesen zeigen:
- Figur 1:: eine perspektivische Ansicht der erfindungsgemäßen Orthese;
- Figur 2:: eine Explosionsdarstellung wesentlicher Teile von Figur 1;
- Figur 3A - 3C:: schematische Darstellungen zur Verdeutlichung der Wirkungsweise des zweiseitig wirkenden Federkraftmechanismus der Orthese von Figur 1;
- Figur 4:: eine perspektivische Explosionsdarstellung der Verriegelungseinrichtung in Form von zwei Klemmringen sowie der Flexions- und Extensionsanschlagträgerscheiben und Anschlagmitnehmereinrichtung;
- Figur 5:: die Anschlagmitnehmereinrichtung von Figur 4 in zusammengebautem Zustand;
- Figur 6:: eine Darstellung der Orthese in der maximalen Flexionsstellung, wobei die Anschlagmitnehmereinrichtung am Flexionsanschlag anschlägt;
- Figur 7:: eine Darstellung der Orthese in der maximalen Extensionsstellung, wobei die Anschlagmitnehmereinrichtung am Extensionsanschlag anschlägt;
- Figur 8A:: eine Seitenansicht des Exzenterelements zum Betätigen der Verriegelungseinrichtung;
- Figur 8B:: eine Draufsicht auf das Exzenterelement von Figur 8A und des damit verbundenen Klemmhebels;
- Figur 9:: eine perspektivische Darstellung der Klemmstange; und
- Figur 10:: eine Explosionsdarstellung der Verriegelungseinrichtung.

Die erfindungsgemäße Orthese wird im folgenden anhand einer Ellbogenorthese detailliert beschrieben. Andere Einsatzmöglichkeiten, beispielsweise als Kniegelenksorthese, sind jedoch ohne weiteres denkbar.

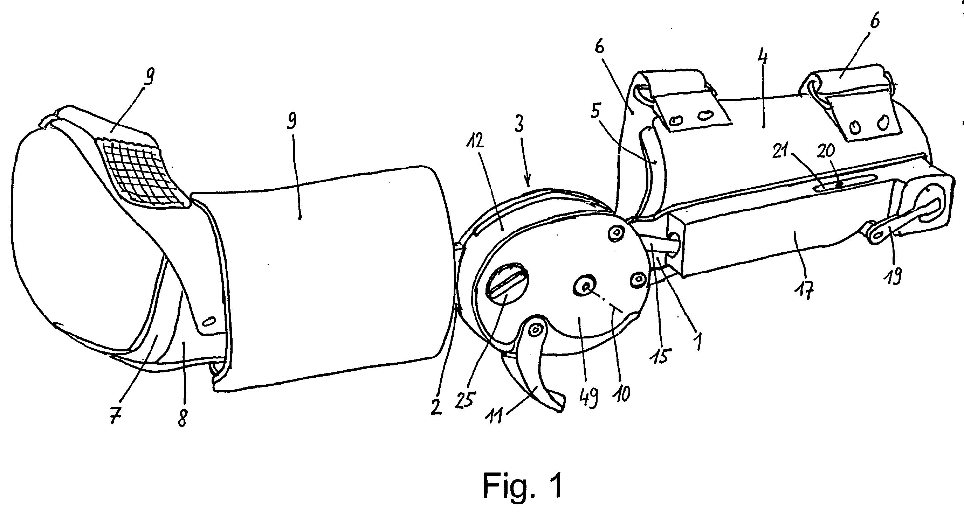

Die in Figur 1 dargestellte Orthese weist eine erste Schiene 1 auf, welche distal, d.h. am Unterarm, befestigt wird, sowie eine zweite Schiene 2, welche proximal, d.h. am Oberarm, befestigt wird und mit der ersten Schiene 1 über ein Schienengelenk 3 gelenkig verbunden ist. Die Orthese wird beim Einsatz als Ellbogenschiene medial angeordnet. Ein lateral angeordnetes Schienenpaar, welches lediglich als "Mitläufer" dient und keinen Federkraftmechanismus aufweist, ist beim Einsatz als Ellbogenschiene nicht erforderlich, könnte jedoch vorgesehen werden, falls dies beispielsweise aus Stabilisierungsgründen erwünscht sein sollte. Wird die Orthese als Kniegelenksorthese eingesetzt, ist es vorteilhaft, auf der gegenüberliegenden Seite des Kniegelenks ebenfalls ein Schienenpaar vorzusehen, das über entsprechende Schalen und/oder Haltebänder mit der dargestellten Orthese und den Körpergliedern verbunden ist.

Zur Befestigung der ersten Schiene 1 am Unterarm ist an der ersten Schiene 1 eine Halbschale 4 aus Kunststoff befestigt, die mit einem Polstermaterial 5, beispielsweise einem Schaumstoffmaterial, ausgekleidet ist. Der Unterarm wird in die Halbschale 4 eingelegt, die mit Befestigungsbändern 6 mit Klettverschluss am Unterarm befestigt wird.

Zur Befestigung der zweiten Schiene 2 am Oberarm weist die zweite Schiene 2 ebenfalls eine mit einem Polstermaterial 7 ausgekleidete Halbschale 8 aus Kunststoff auf, die mit Befestigungsbändern 9 mit Klettverschluss am Oberarm befestigt wird.

Die erste Schiene 1 ist mit der zweiten Schiene 2 gelenkig verbunden, wobei die Schwenkachse mit dem Bezugszeichen 10 bezeichnet ist. Weiterhin weist die Orthese einen doppelt wirkenden Federkraftmechanismus auf, der auf die Orthese eine Vorspannkraft sowohl in Flexions- als auch in Extensionsrichtung aufbringt. Die Umschaltung der Vorspannkraft von Flexionsrichtung in Extensionsrichtung und umgekehrt erfolgt hierbei automatisch ab einem bestimmten, einstellbaren Schwenkwinkel, wie später noch näher ausgeführt wird. Weiterhin weist die Orthese eine Mechanik auf, die es gestattet, einen Flexionsanschlag, der zur Begrenzung des Schwenkbereichs in Flexionsrichtung dient, sowie einen Extensionsanschlag, der zur Begrenzung des Schwenkbereichs in Extensionsrichtung dient, auf einfache, schnelle und sehr exakte Weise stufenlos zu verstellen. Die Verstellung erfolgt hierbei durch eine Schwenkbewegung der ersten Schiene 1 gegenüber der zweiten Schiene 2 bis zu der gewünschten Schwenkbereichsgrenze, wobei der Flexions- bzw. Extensionsanschlag bis zu der jeweiligen Schwenkbereichsgrenze mitgenommen wird und dort mittels einer einfach zu betätigenden Verriegelungseinrichtung, die über einen Klemmhebel betätigt wird, arretiert werden kann. Auch dies wird im Folgenden noch näher erläutert.

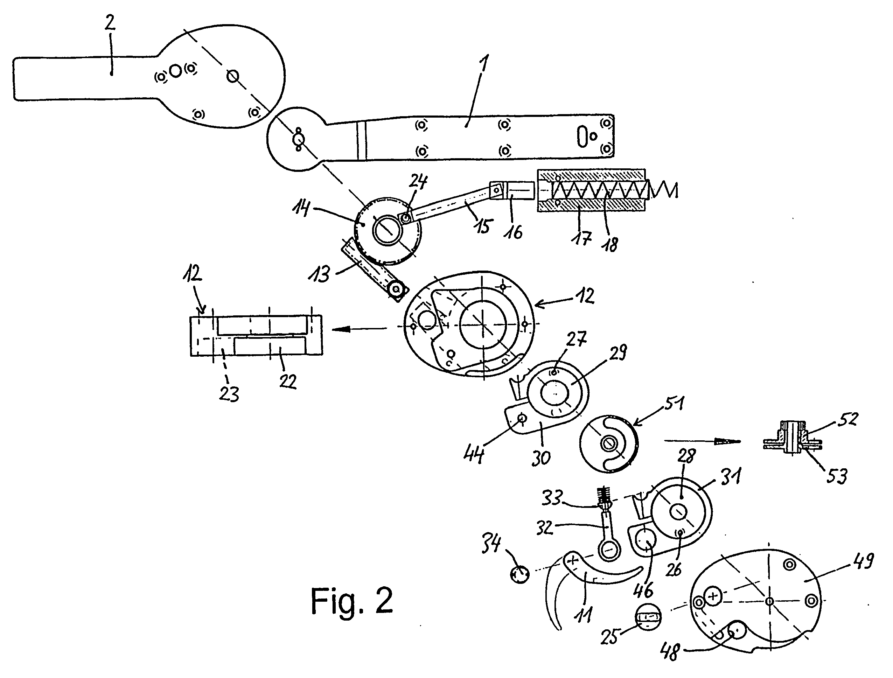

Wie aus Figur 2 ersichtlich, bildet die proximal anzuordnende zweite Schiene 2 den rückseitigen Abschluss eines Gehäuses 12, das in Figur 2 sowohl in Seitendarstellung als auch links daneben in einem mittigen Horizontalschnitt dargestellt ist. Unmittelbar neben der zweiten Schiene 2 befindet sich die distal anzuordnende erste Schiene 1. An diese erste Schiene 1 schließt sich in Achsrichtung der doppelt wirkende Federkraftmechanismus an, der aus einem Schnecken-/ Schneckenradantrieb mit einer Schnecke 13 und einem Schneckenrad 14, einer gelenkig mit dem Schneckenrad 14 verbundenen Pleuelstange 15, einem an der ersten Schiene 1 befestigten Federgehäuse 17 sowie einer im Federgehäuse 17 geführten Druckfeder 18 besteht, die auf einen Kolben 16 und damit auf die Pleuelstange 15 eine Vorspannkraft ausübt. Das bezüglich des Kolbens 16 gegenüberliegende Ende der Druckfeder 18 kann mittels einer ausklappbaren Kurbel 19 (Figur 1), die mit einem nicht dargestellten Winkelgetriebe und einem Stellkolben in Verbindung ist, längs des Federgehäuses 17 verschoben werden, um die Vorspannung der Druckfeder 18 auf den gewünschten Wert einzustellen. Die eingestellte Position des rückseitigen Endes der Druckfeder 18 und damit die Höhe der Vorspannkraft wird mittels eines Anzeigestifts 20 (Figur 1) angezeigt, der mit dem rückwärtigen Stellkolben in Verbindung steht und durch einen Schlitz 21 im Federgehäuse 17 hindurch sichtbar ist.

Das Schneckenrad 14 ist drehbar in einem rückwärtigen Hohlraum 23 des Gehäuses 12 gelagert. Die Schnecke 13 ist ebenfalls in einem lediglich gestrichelt eingezeichneten rückseitigen Hohlraum 23 derart gelagert, dass sie um ihre Längsachse drehbar ist. Da das Schneckenrad 14 mit der Schnecke 13 in Eingriff ist und es sich um ein selbsthemmendes Getriebe handelt, bleibt die eingestellte Drehposition des Schneckenrads 14 und damit die Lage eines Anlenkpunktes 24 der Pleuelstange 15 relativ zum Gehäuse 12 und relativ zur zweiten Schiene 2, die mit dem Gehäuse 12 fest verbunden ist, erhalten, solange nicht die Schnecke 13 gedreht wird. Die Lage des Anlenkpunktes 24 relativ zur zweiten Schiene 2 bestimmt den Totpunkt, d.h. den Schwenkwinkel, ab dem der Federkraftmechanismus die Orthese von Flexion auf Extension umschaltet und umgekehrt. Dies wird später noch näher erläutert.

Die Einstellung des Schnecken-/ Schneckenradantriebs erfolgt durch Drehen eines Drehknopfs 25 (Figur 1), der über ein nicht näher dargestelltes Getriebe mit der Schnecke 13 in Wirkverbindung ist. Beim Drehen des Drehknopfs 25 wird die Schnecke 13 um ihre Längsachse gedreht, wodurch das Schneckenrad 14 ebenfalls gedreht wird und der Anlenkpunkt 24 seine Winkelposition relativ zur zweiten Schiene 2 verändert.

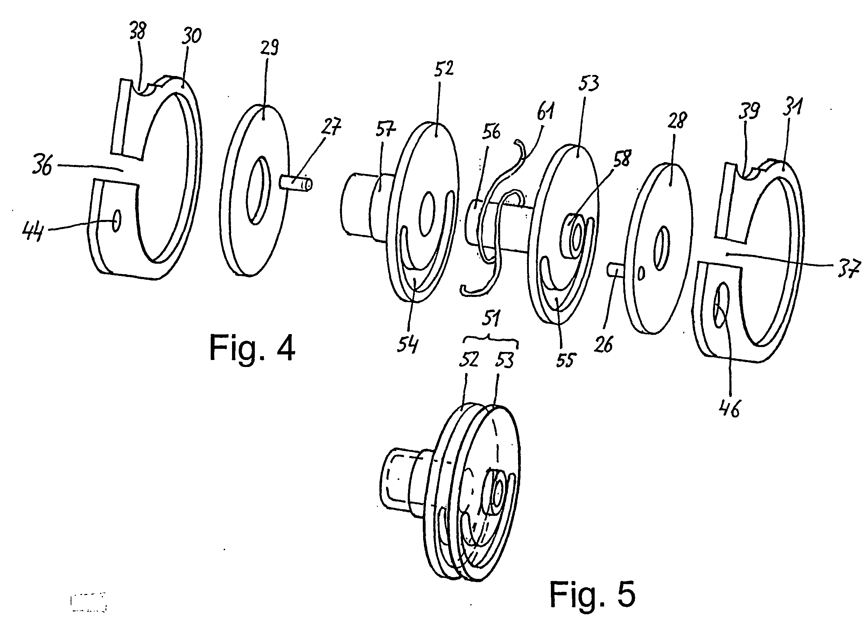

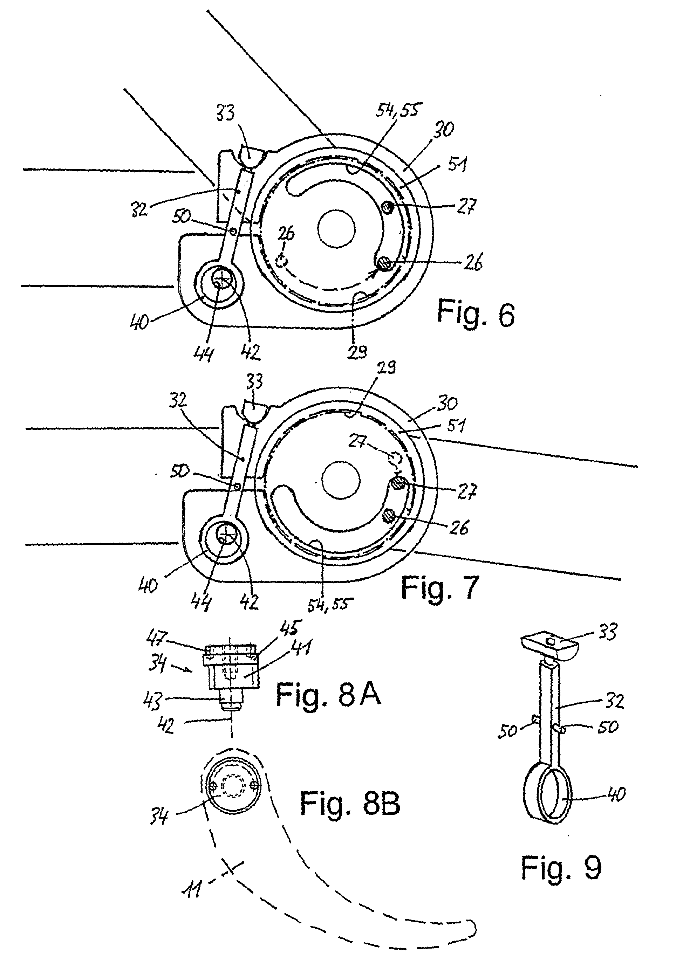

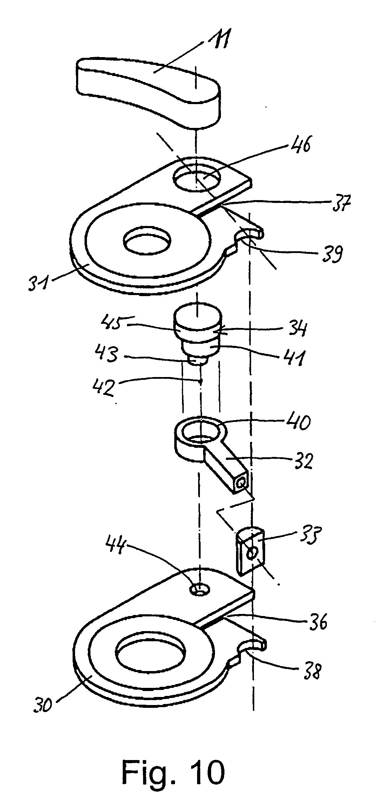

Der freie Schwenkbereich der ersten Schiene 1 gegenüber der zweiten Schiene 2 wird in Flexionsrichtung durch einen Flexionsanschlag 26 und in Extensionsrichtung durch einen Extensionsanschlag 27 (Figuren 2, 4, 6 und 7) in Form von Querstiften begrenzt. Wie insbesondere aus den Figuren 2 und 4 ersichtlich, sind der Flexionsanschlag 26 an einer Flexionsanschlagträgerscheibe 28 und der Extensionsanschlag 27 an einer Extensionsanschlagträgerscheibe 29 außermittig befestigt und stehen in Querrichtung, d.h. parallel zur Schwenkachse 10, vor. Die Flexions- und Extensionsanschlagträgerscheibe 28, 29 sind in einem nicht verriegelten Zustand unabhängig voneinander um die Schwenkachse 10 drehbar, so dass die Winkelposition des Flexionsanschlags 26 und Extensionsanschlags 27 relativ zum Gehäuse 12 und damit zur zweiten Schiene 2 verändert und in gewünschter Weise eingestellt werden kann. Befinden sich der Flexionsanschlag 26 und Extensionsanschlag 27 an der gewünschten Stelle, werden die Flexions- und Extensionsanschlagträgerscheiben 28, 29 relativ zum Gehäuse 12 und damit zur zweiten Schiene 2 arretiert. Diese Arretierung erfolgt mittels einer in den Figuren 4 und 10 näher dargestellten Verriegelungseinrichtung, die aus zwei die Flexions- und Extensionsanschlagträgerscheibe 28, 29 umgebenden Klemmringen 30, 31, einer Klemmstange 32 mit Querjoch 33, einem Exzenterelement 34 (Figuren 8A, 8B) und dem manuell betätigbaren Klemmhebel 11 besteht, der mit dem Exzenterelement 34 drehfest verbunden ist. Die Verriegelungseinrichtung befindet sich mit Ausnahme des Klemmhebels 11 in einem vorderen Hohlraum 35 (Figur 2) des Gehäuses 12.

Die Klemmringe 30, 31 der Verriegelungseinrichtung haben einen Innendurchmesser, der im Wesentlichen dem Außendurchmesser der Flexions- und Extensionsanschlagträgerscheiben 28, 29 entspricht, so dass sie mit geringem Spiel auf die Flexions- bzw. Extensionsanschlagträgerscheibe 28, 29 aufgesetzt werden können. Im aufgesetzten Zustand befinden sich die Klemmringe 30, 31 somit in derselben Ebene wie die Flexions- bzw. Extensionsanschlagträgerscheibe 28, 29. Die Dicke der Klemmringe 30, 31 entspricht in etwa derjenigen der Flexions- bzw. Extensionsanschlagträgerscheibe 28, 29. Weiterhin weisen die Klemmringe 30, 31 jeweils einen durchgehenden Schlitz 36 bzw. 37 auf, so dass die Enden der Klemmringe 30, 31 zusammengezogen oder voneinander wegbewegt werden können. Werden die Klemmringe 30, 31 zusammengezogen, sitzen sie auf der Umfangsfläche der Flexions- bzw. Extensionsanschlagträgerscheibe 28, 29 fest auf, so dass sich diese nicht mehr relativ zu den Klemmringen 30, 31 drehen können. Werden die Klemmringe 30, 31 gespreizt, können sich die Flexions- bzw. Extensionsanschlagträgerscheibe 28, 29 frei drehen. In einer Zwischenstellung, in welcher die Klemmringe 30, 31 weder zusammengezogen noch aktiv gespreizt werden, liegen die Klemmringe 30, 31 aufgrund ihrer Eigenspannung nur geringfügig auf der Flexions- bzw. Extensionsanschlagträgerscheibe 28, 29 auf, so dass die Flexions- bzw. Extensionsanschlagträgerscheibe 28, 29 unter Aufbringung einer geringen Kraft gedreht werden können.

Das Zusammenziehen der Klemmringe 30, 31 erfolgt über die in Figur 9 in Alleinstellung dargestellte Klemmstange 32, welche das Querjoch 33 mit dem Exzenterelement 34 (Figur 8A) verbindet. Die Klemmstange 32 liegt im zusammengebauten Zustand der Orthese zwischen den Klemmringen 30, 31, die zueinander einen gewissen Abstand aufweisen. Das Querjoch 33 übergreift beide Klemmringe 30, 31 von oben und liegt in den halbkreisförmigen Vertiefungen 38, 39, die im oberen Ende der Klemmringe 30, 31 ausgebildet sind. Am unteren Ende weist die Klemmstange 32 eine ringförmige Buchse 40 auf. Diese Buchse 40 dient zur Aufnahme einer Exzenterfläche 41 des Exzenterelements 34, die exentrisch zur Drehachse 42 des Exzenterelements 34 angeordnet ist. Im Übrigen ist das Exzenterelement 34 im Bereich seines Endes 43, das sich in Figur 8A unten befindet, in einer Durchgangsbohrung 44 des hinteren Klemmrings 30 gelagert. Eine Lagerfläche 45 des Exzenterelements 34, die sich in Figur 8A oben befindet, sitzt in einer Durchgangsbohrung 46 des vorderen Klemmrings 37, die zur Durchgangsbohrung 44 des hinteren Klemmrings 30 fluchtet. Weiterhin weist das Exzenterelement 34 eine vordere Lagerfläche 47 auf, die in einer Durchgangsbohrung 48 eines vorderen Gehäusedeckels 49 sitzt. Das Exzenterelement 34 ist somit unverschiebbar, jedoch um seine eigene Drehachse 42 drehbar im Gehäuse 12 der Orthese gelagert und hält gleichzeitig die Klemmringe 30, 31 an Ort und Stelle, so dass diese relativ zum Gehäuse 12 und damit auch relativ zur zweiten Schiene 2 arretiert sind.

Wird das Exzenterelement 34 mittels des Klemmhebels 11 derart gedreht, dass sich die Exzenterfläche 41 nach unten bewegt, wird die Klemmstange 32 ebenfalls nach unten gezogen und zieht das obere Ende der Klemmringe 30, 31 mittels des Querjochs 33 ebenfalls nach unten, d.h. in Richtung zum unteren Ende der Klemmringe 30, 31. Hierdurch werden die Flexions- und Extensionsanschlagträgerscheiben 28, 29 drehfest arretiert. Wird der Klemmhebel 35 nach unten geschwenkt (Figur 1), bewegt sich die Exzenterfläche 41 des Exzenterelemts 34 nach oben, wodurch die Zugspannung der Klemmstange 32 aufgehoben wird. Die Flexions- und Extensionsanschlagträgerscheiben 28, 29 können nun in einer später noch näher beschriebenen Weise mit geringem Kraftaufwand gedreht werden. Wird der Klemmhebel 35 weiter (in Figur 1 nach links) gedreht, wird die Klemmstange 32 weiter nach oben geschoben, wobei die Klemmringe 30, 31 mittels Querstifte 60, welche an der Klemmstange 32 im Bereich der Schlitze 36, 37 angeordnet sind, gespreizt werden. Hierdurch wird der Reibschluss zwischen den Klemmringen 30, 31 und den Flexions- bzw. Extensionsanschlagträgerscheiben 28, 29 vollkommen aufgehoben, so dass sich die Flexions- bzw. Extensionsanschlagträgerscheibe 28, 29 ohne jede Behinderung bewegen lassen.

Das Einstellen des Flexions- und Extensionsanschlags 26, 27 erfolgt mittels einer Anschlagmitnehmereinrichtung 51, die in Figur 4 in Einzeldarstellung und in Figur 5 in zusammengebautem Zustand dargestellt ist. Die Anschlagmitnehmereinrichtung 51 ist drehfest mit der ersten Schiene 1 verbunden und schwenkt mit dieser mit. Die Anschlagmitnehmereinrichtung 51 besteht aus zwei Anschlagmitnehmerscheiben 52, 53, die jeweils einen bogenförmig um die Schwenkachse 10 herumgeführten Längsschlitz 54, 55 aufweisen. Die Längsschlitze 54, 55 erstrecken sich in Umfangsrichtung über einen Winkel von etwa 180 Grad. Die vordere Anschlagmitnehmerscheibe 53 weist einen mittigen, rohrförmigen Lagerfortsatz 56 auf, der in einen rohrförmigen Lagerfortsatz 57 größeren Durchmessers der hinteren Anschlagmitnehmerscheibe 52 eingesteckt werden kann, wie aus Figur 5 ersichtlich ist. Im montierten Zustand sind die beiden Anschlagmitnehmerscheiben 52, 53 drehfest miteinander verbunden, wobei sie zueinander parallel und mit einem gewissen Abstand angeordnet sind. Die beiden Längsschlitze 54, 55 sind fluchtend zueinander ausgerichtet.

Die Extensionsanschlagträgerscheibe 29 ist auf dem hinteren Lagerfortsatz 57 in unmittelbarer Nachbarschaft der hinteren Anschlagmitnehmerscheibe 52 gelagert, wobei sich der Extensionsanschlag 27 in den Längsschlitz 54 hineinerstreckt. Die Flexionsanschlagträgerscheibe 28 ist auf einem vorderen Lagerbund 58 der vorderen Anschlagmitnehmerscheibe 53 in deren unmittelbarer Nachbarschaft gelagert, wobei der Flexionsanschlag 26 in den Längsschlitz 55 hineinragt. Die Flexions- und Extensionsanschläge 26, 27 besitzen eine derartige Länge, dass sie sich vollständig durch den benachbarten Längsschlitz 55 bzw. 54 hindurch erstrecken und sogar noch etwas in den entfernten Längsschlitz 54 bzw. 55 hineinragen. Der Flexionsanschlag 26 und Extensionsanschlag 27 überbrücken somit den Zwischenraum zwischen den beiden Anschlagmitnehmerscheiben 52, 53. Auf Grund dieser Anordnung ist es möglich, im Zwischenraum zwischen den Anschlagmitnehmerscheiben 52, 53 eine Rückführfeder 61 (Figur 4) vorzusehen, die sowohl am Flexionsanschlag 26 als auch am Extensionsanschlag 27 angreift und versucht, diese in eine Ausgangsposition zurückzudrängen, d.h. die Flexionsanschlagträgerscheibe 28 und Extensionsanschlagträgerscheibe 29 in eine Ausgangsposition zurückzudrehen, in der der Flexionsanschlag 26 und Extensionsanschlag 27 um 180 Grad zueinander versetzt sind. Diese Ausgangsposition ist in den Figuren 6 und 7 gestrichelt eingezeichnet. In dieser Ausgangslage liegt der Flexionsanschlag 26 an einem Ende der Längsschlitze 54, 55 und der Extensionsanschlag 27 am anderen Ende der Längsschlitze 54, 55 an. Um den Flexionsanschlag 26 und Extensionsanschlag 27 in ihre um 180 Grad versetzten Ausgangslagen zurückzuführen, ist es erforderlich, dass die Klemmringe 30, 31 gespreizt und damit von der Flexionsanschlagträgerscheibe 28 und Extensionsanschlagträgerscheibe 29 abgehoben werden. Dieses Spreizen erfolgt, wie bereits ausgeführt, dadurch, dass der Klemmhebel 11 maximal nach unten bzw. links (gemäß Figur 1) geschwenkt wird, wodurch das Exzenterelement 34 die Klemmstange 32 anhebt und die Querstifte 60 der Klemmstange 32 die Schlitze 36, 37 erweitert.

Um den Flexionsanschlag 26 und Extensionsanschlag 27 an der gewünschten Stelle zu positionieren, wird zunächst der Klemmhebel 11 in eine neutrale Stellung geschwenkt, wodurch die Klemmstange 32 weder Zug noch Druck auf die Klemmringe 30, 31 ausübt. Die Klemmringe 30, 31 drücken jetzt auf Grund ihrer Eigenspannung ein gewisses Maß auf die Flexions- und Extensionsanschlagträgerscheiben 28, 29, so dass diese zwar noch gedreht werden können, sich jedoch nicht mehr selbständig auf Grund der Federkraft der nicht näher dargestellten Rückführfeder in ihre Ausgangslage zurückdrehen. Wird nun die erste Schiene 1 zusammen mit den damit drehfest gekoppelten Anschlagmitnehmerscheiben 52, 53 von einer Extensionsstellung, beispielsweise derjenigen, die in Figur 7 gezeigt ist, bis zu einer gewünschten maximalen Flexionsstellung gemäß Figur 6 geschwenkt, wird der Flexionsanschlag 26 von den Anschlagmitnehmerscheiben 52, 53 mitgenommen, wie durch den Pfeil 59 in Figur 6 dargestellt, da der Flexionsanschlag 26 am Ende der Längsschlitze 54, 55 anschlägt. Der Extensionsanschlag 27 bleibt bei dieser Schwenkbewegung in Flexionsrichtung vorerst an Ort und Stelle, da er sich im Schlitzbereich befindet, ohne an einem der beiden Schlitzenden anzuschlagen.

Wird nun der erste Anschlag 1 von der in Figur 6 gezeigten maximalen Flexionsstellung in die gewünschte maximale Extensionsstellung zurückgeschwenkt, wie in Figur 7 dargestellt, wird der Extensionsanschlag 27 von den Anschlagmitnehmerscheiben 52, 53 in Richtung des Pfeils 60 mitgenommen, da der Extensionsanschlag 27 am Ende der Längsschlitze 54, 55 anschlägt. Der Flexionsanschlag 26 bleibt bei dieser Schwenkbewegung in Extensionsrichtung an Ort und Stelle, da er sich im freien Schlitzbereich befindet, an keinem Ende der Längsschlitze 54, 55 anschlägt und darüber hinaus durch die geringe Eigenklemmkraft des die Flexionsanschlagträgerscheibe 28 umgebenden Klemmrings 31 am Zurückdrehen in die Ausgangslage geändert wird.

In der Stellung, die in Figur 7 gezeigt ist, befinden sich somit sowohl der Flexionsanschlag 26 als auch der Extensionsanschlag 27 an den gewünschten Stellen zur Begrenzung des Schwenkbereichs in Flexions- bzw. Extensionsrichtung.

Die Position des Flexionsanschlags 26 und Extensionsanschlags 27 wird nun arretiert, indem der Klemmhebel 11 in seine Klemmstellung, d.h. in Figur 1 nach rechts, zurückgeschwenkt wird. Hierdurch wird die Klemmstange 32 über das Exzenterelement 34 nach unten gezogen und die beiden Klemmringe 30, 31 zusammengezogen. Die Flexions-und Extensionsanschlagträgerscheiben 28, 29 sitzen damit fest innerhalb der Klemmringe 30, 31 und sind drehfest mit dem Gehäuse 12 und damit auch mit der zweiten Schiene 2 verbunden. Nach dieser Verriegelung des Flexionsanschlags 26 und Extensionsanschlags 27 kann die erste Schiene 1 in Flexionsrichtung nur mehr soweit geschwenkt werden, bis das Ende der Längsschlitze 54, 55 am Flexionsanschlag 26 anschlägt, wie in Figur 6 gezeigt. Weiterhin kann die erste Schiene 1 in Extensionsrichtung nur mehr soweit geschwenkt werden, bis das Ende der Längsschlitze 54, 55 am Extensionsanschlag 27 anschlägt, wie in Figur 7 gezeigt.

Sollen die Schwenkbereichsgrenzen nachgestellt werden, so ist es lediglich erforderlich, den Klemmhebel 11 zu lösen, worauf der Flexions- und Extensionsanschlag 26, 27 zu den neuen Schwenkbereichsgrenzen wieder mitgenommen werden können.

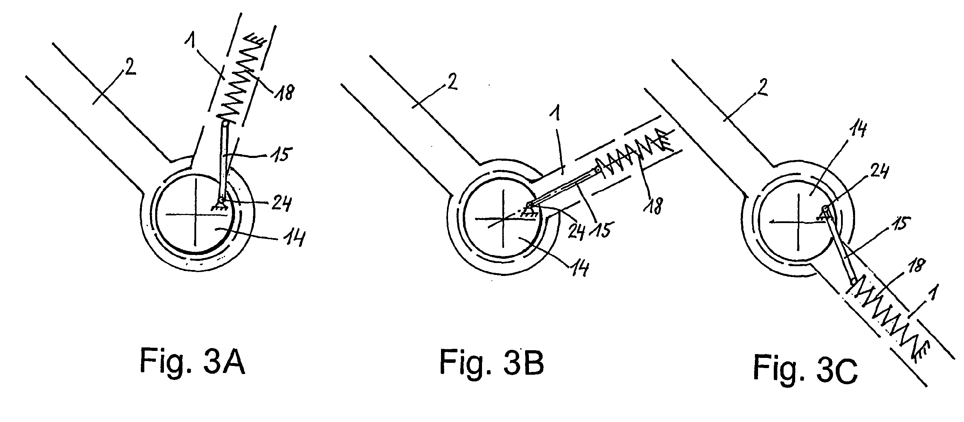

Im folgenden wird anhand der Figuren 3A - 3C die Wirkung des doppelt wirkenden Federkraftmechanismus näher erläutert. Der besseren Übersicht wegen ist in diesen schematischen Zeichnungen im Bereich des Schienengelenks 3 lediglich das Schneckenrad 14 mit dem Anlenkpunkt 24 der Pleuelstange 15 eingezeichnet.

In der in Figur 3 Autotransporter gezeigten Stellung befindet sich die Orthese im Flexionsbereich, in dem die erste Schiene 1 auf Grund der Schubkraft der Druckfeder 18, die über die Pleuelstange 15 auf den außermittig am Schneckenrad 14 angeordneten Anlenkpunkt 24 übertragen wird, in Flexionsrichtung, d.h. im Gegenuhrzeigersinn, gedrängt wird.

Streckt nun der Patient seinen Unterarm und schwenkt die erste Schiene 1 im Uhrzeigersinn, muss er zunächst eine zunehmende Vorspannkraft der Druckfeder 18 überwinden, da bei dieser Bewegung die Druckfeder 18 zusammengedrückt wird.

Das Zusammendrücken der Druckfeder 18 erfolgt solange, bis der in Figur 3B gezeigte Schwenkwinkel erreicht wird. Bei diesem Schwenkwinkel, der als Totpunkt bezeichnet wird, befindet sich die Pleuelstange 15 in gerader Linie von der Druckfeder 18 zur Schwenkachse 10. Die Schubkraft der Druckfeder 18 geht somit durch die Schwenkachse 10, so dass kein Drehmoment auf die erste Schiene 1 übertragen wird.

Streckt der Patient seinen Unterarm weiter und damit die erste Schiene 1 über den Totpunkt hinaus in Extensionsrichtung, wie in Figur 3C dargestellt, beginnt sofort wieder ein Drehmoment auf die erste Schiene 1 zu wirken, da nun die Schubkraft der Druckfeder 18 nicht mehr durch die Schwenkachse 10, sondern seitlich an dieser vorbeigeht.

Je nachdem, in welchem Schwenkwinkelbereich bezüglich des Totpunkts sich die erste Schiene 1 relativ zur zweiten Schiene 2 befindet, übt die Druckfeder 18 somit ein Drehmoment in Flexions- oder Extensionsrichtung aus.

Bezüglich des doppelt wirkenden Federkraftmechanismus wird auch auf die deutsche Patentanmeldung Nr. 199 04 554.2 verwiesen, auf deren Offenbarungsgehalt Bezug genommen wird.

Alternativ zu dem beschriebenen Ausführungsbeispiel ist es ohne weiteres möglich, die Verriegelungseinrichtung für die Flexions- und Extensionsanschlagträgerscheiben 28, 29 nicht reibschlüssig als Klemmeinrichtung, sondern kraftschlüssig durch Ausgestaltung der Flexions- und Extensionsanschlagträgerscheiben 28, 29 als Zahnräder auszubilden. Weiterhin ist es auch möglich, die Klemmflächen zwischen Flexions- und Extensionsanschlagträgerscheiben 28, 29 und den Klemmringen 30, 31 mit reibungserhöhenden Beschichtungen zu versehen.

## Patentansprüche

1. Orthese zur Reduktion von Streck- und/oder Beugedefiziten eines ersten Körperglieds gegenüber einem mit dem ersten Körperglied gelenkig verbundenen zweiten Körperglied, mit
- einer ersten Schiene (1), die am ersten Körperglied befestigbar ist,
- einer zweiten Schiene (2), die am zweiten Körperglied befestigbar ist,
- einem Schienengelenk (3), das die erste Schiene (1) und zweite Schiene (2) gelenkig miteinander verbindet,
- einem zwischen der ersten Schiene (1) und zweiten Schiene (2) wirkenden Federkraftmechanismus, um die erste Schiene (1) gegenüber der zweiten Schiene (2) zu verschwenken,
- einem relativ zur zweiten Schiene (2) arretierbaren Flexionsanschlag (26) zur Begrenzung des Schwenkbereichs in Flexionsrichtung,
- einem relativ zur zweiten Schiene (2) arretierbaren Extensionsanschlag (27) zur Begrenzung des Schwenkbereichs in Extensionsrichtung, **dadurch gekennzeichnet,**
- **dass** der Flexionsanschlag (26) auf einer um die Schwenkachse (10) des Schienengelenks (3) drehbaren Flexionsanschlagträgerscheibe (28) angeordnet ist,
- **dass** der Extensionsanschlag (27) auf einer um die Schwenkachse (10) des Schienengelenks (3) drehbaren Extensionsanschlagträgerscheibe (29) angeordnet ist,
- **dass** die Flexionsanschlagträgerscheibe (28) und Extensionsanschlagträgerscheibe (29) mit einer lösbaren Verriegelungseinrichtung in Wirkverbindung sind, um ein Drehen der Flexionsanschlagträgerscheibe (28) und Extensionsanschlagträgerscheibe (29) um die Schwenkachse (10) zu blockieren oder zu ermöglichen,
- **dass** eine mit der ersten Schiene (1) drehfest gekoppelte und zusammen mit dieser um die Schwenkachse (10) drehbare Anschlagmitnehmereinrichtung (51) vorgesehen ist, mit der bei gelöster Verriegelungseinrichtung der Flexionsanschlag (26) durch Schwenken der ersten Schiene (1) bis zur gewünschten Flexionsbereichsgrenze und der Extensionsanschlag (27) durch Schwenken der ersten Schiene (1) bis zur gewünschten Extensionsschwenkbereichsgrenze bewegbar sind,
- und **dass** durch Verriegeln der Verriegelungseinrichtung der Flexionsanschlag (26) an der Flexionsschwenkbereichsgrenze und der Extensionsanschlag (27) an der Extensionsschwenkbereichsgrenze arretierbar sind.

2. Orthese nach Anspruch 1, **dadurch gekennzeichnet, dass** die mit der ersten Schiene (1) drehfest gekoppelte Anschlagmitnehmereinrichtung (51) auch als Schwenkbereichbegrenzungsmittel ausgebildet ist, das an der Flexionsschwenkbereichsgrenze am Flexionsanschlag (26) und an der Extensionsschwenkbereichsgrenze am Extensionsanschlag (27) anschlägt.

3. Orthese nach Anspruch 1 oder 2, **dadurch gekennzeichnet, dass** die Anschlagmitnehmereinrichtung (51) aus mindestens einer Anschlagmitnehmerscheibe (52, 53) mit einem bogenförmig um die Schwenkachse (10) herumgeführten Längsschlitz (54, 55) besteht, in den der Flexionsanschlag (26) und Extensionsanschlag (27) hineinragen.

4. Orthese nach einem der vorhergehenden Ansprüche, **dadurch gekennzeichnet, dass** eine an der Flexionsanschlagträgerscheibe (28) und Extensionsanschlagträgerscheibe (29) angreifende Rückführfeder (61) vorgesehen ist, um den Flexionsanschlag (26) und Extensionsanschlag (27) bei gelöster Verriegelungseinrichtung in ihre Ausgangslagen zurückzuführen.

5. Orthese nach einem der vorhergehenden Ansprüche, **dadurch gekennzeichnet, dass** die Verriegelungseinrichtung aus einer an der zweiten Schiene (2) gehalterten Klemmeinrichtung besteht, welche die Flexionsanschlagträgerscheibe (28) und Extensionsanschlagträgerscheibe (29) übergreift und mittels einer gemeinsamen Klemmbetätigungseinrichtung zwischen einer Klemmstellung und einer Freigabestellung bewegbar ist.

6. Orthese nach einem der vorhergehenden Ansprüche, **dadurch gekennzeichnet, dass** die Klemmeinrichtung aus zwei Klemmringen (30, 31) besteht, welche die Flexionsanschlagträgerscheibe (28) und Extensionsanschlagträgerscheibe (29) an ihrer äußeren Umfangsfläche mit engem Spiel übergreifen und in der Klemmstellung ein Drehen der Flexionsanschlagträgerscheibe (28) und Extensionsanschlagträgerscheibe (29) verhindern.

## Claims

1. Orthesis for reducing extension and/or bending deficits of a first extremity relative to a second extremity which is hinged to the first extremity, with
- a first rail (1) which can be attached to the first extremity,
- a second rail (2) which can be attached to the second extremity,
- a rail hinge (3) which hinges the first rail (1) and the second rail (2) to one another,
- a spring force mechanism which acts between the first rail (1) and the second rail (2) in order to swivel the first rail (1) relative to the second rail (2),
- a flexion stop (26) which can be locked relative to the second rail (2) for limiting the swivelling range in the flexion direction,
- an extension stop (27) which can be locked relative to the second rail (2) for limiting the swivelling range in the extension direction, **characterized in that**
- the flexion stop (26) is located on a flexion stop carrier disk (28) which can be turned around the swivelling axis (10) of the rail hinge (3),
- the extension stop (27) is located on the extension stop carrier disk (29) which can be turned around the swivelling axis (10) of the rail hinge (3),
- the flexion stop carrier disk (28) and extension stop carrier disk (29) are dynamically connected to a releasable locking means in order to block or enable the rotation of the flexion stop carrier disk (28) and extension stop carrier disk (29) around the swivelling axis (10),
- there is a stop driver means (51) which is coupled torsionally strong to the first rail (1), which can turn together with it around the swivelling axis (10), and with which with the locking means released the flexion stop (26) can be moved by swivelling the first rail (1) as far as the desired limit of the flexion range and the extension stop (27) can be moved by swivelling the first rail (1) as far as the desired limit of the extension swivelling range, and
- by locking the locking means the flexion stop (26) can be locked at the limit of the flexion swivelling range and the extension stop (27) can be locked at the limit of the extension swivelling range.

2. Orthesis as claimed in claim 1, wherein the stop driver means (51) which is coupled torsionally strong to the first rail (1) is also made as a swivelling range limitation means which at the limit of the flexion swivelling range strikes the flexion stop (26) and at the limit of the extension swivelling range strikes the extension stop (27).

3. Orthesis as claimed in claim 1 or 2, wherein the stop driver means (51) consists of at least one stop driver disk (52, 53) with a lengthwise slot (54, 55) which is routed in an arc shape around the swivelling axis (10) and into which the flexion stop (26) and extension stop (27) project.

4. Orthesis as claimed in one of the preceding claims, wherein there is a return spring (61) which engages the flexion stop carrier disk (28) and the extension stop carrier disk (29) in order to return the flexion stop (26) and extension stop (27) to their initial location when the locking means has been released.

5. Orthesis as claimed in one of the preceding claims, wherein the locking means consists of a clamping means which is held on the second rail (2), which extends over the flexion stop carrier disk (28) and the extension stop carrier disk (29), and which can be moved by a common clamp actuating means between the clamp position and the release position.

6. Orthesis as claimed in one of the preceding claims, wherein the clamp means feasibly consists of two clamping rings (30, 31) which extend over the flexion stop carrier disk (28) and the extension stop carrier disk (29) on their outer peripheral surface with narrow play and in the clamped position prevent rotation of the flexion stop carrier disk (28) and the extension stop carrier disk (29).

## Revendications

1. Orthèse pour réduire des déficits d'extension et/ou de flexion d'un premier membre corporel par rapport à un second membre corporel relié en articulation au premier membre corporel, comprenant :
- une première tringle (1) susceptible d'être fixée sur le premier membre corporel,
- une seconde tringle (2) susceptible d'être fixée sur le second membre corporel,
- une articulation de tringle (3) qui relie en articulation la première tringle (1) et la seconde tringle (2) l'une à l'autre,
- un mécanisme à force élastique agissant entre la première tringle (1) et la seconde tringle (2) pour faire pivoter la première tringle (1) par rapport à la seconde tringle (2),
- une butée de flexion (26) susceptible d'être arrêtée par rapport à la seconde tringle (2) et destinée à limiter la zone de pivotement en direction de flexion,
- une butée d'extension (27) susceptible d'être arrêtée par rapport à la seconde tringle (2) et destinée à limiter la zone de pivotement en direction d'extension,
**caractérisée en ce que**
- la butée de flexion (26) est agencée sur un disque porteur de butée de flexion (28) mobile en rotation autour de l'axe de pivotement (10) de l'articulation de tringle (3),
- la butée d'extension (27) est agencée sur un disque porteur de butée d'extension (29) mobile en rotation autour de l'axe de pivotement (10) de l'articulation de tringle (3),
- le disque porteur de butée de flexion (28) et le disque porteur de butée d'extension (29) sont en liaison d'action avec un dispositif de verrouillage libérable pour bloquer ou permettre une rotation du disque porteur de butée de flexion (28) et du disque porteur de butée d'extension (29) autour de l'axe de pivotement (10),
- il est prévu un dispositif entraîneur de butée (51) couplé solidairement en rotation à la première tringle (1) et mobile conjointement avec celle-ci en rotation autour de l'axe de pivotement (10), dispositif par lequel, le dispositif de verrouillage de la butée de flexion (26) étant libéré, la butée de flexion (26) est mobile par pivotement de la première tringle (1) jusqu'à la limite désirée de la zone de flexion et la butée d'extension (27) est mobile par pivotement de la première tringle (1) jusqu'à la limite désirée de la zone de pivotement d'extension,
- et par verrouillage du dispositif de verrouillage, la butée de flexion (26) est susceptible d'être arrêtée à la limite de la zone de pivotement de flexion et la butée d'extension (27) est susceptible d'être arrêtée à la limite de la zone de pivotement d'extension.

2. Orthèse selon la revendication 1, **caractérisée en ce que** le dispositif entraîneur de butée (51) couplé solidairement en rotation à la première tringle (1) est réalisé également à titre de moyen de limitation de la zone de pivotement qui vient buter contre la butée de flexion (26) à la limite de la zone de pivotement de flexion et contre la butée d'extension (27) à la limite de la zone de pivotement d'extension.

3. Orthèse selon la revendication 1 ou 2, **caractérisée en ce que** le dispositif entraîneur de butée (51) est constitué par au moins un disque entraîneur de butée (52, 53) pourvu d'une fente oblongue (54, 55) menée en forme d'arc autour de l'axe de pivotement (10), fente dans laquelle pénètrent la butée de flexion (26) et la butée d'extension (27).

4. Orthèse selon l'une des revendications précédentes, **caractérisée en ce qu'**il est prévu un ressort de rappel (61) qui attaque le disque porteur de butée de flexion (28) et le disque porteur de butée d'extension (29) pour rappeler la butée de flexion (26) et la butée d'extension (27) dans leurs positions initiales lorsque le dispositif de verrouillage est libéré.

5. Orthèse selon l'une des revendications précédentes, **caractérisée en ce que** le dispositif de verrouillage est constitué par un dispositif de serrage monté sur la seconde tringle (2), lequel coiffe le disque porteur de butée de flexion (28) et le disque porteur de butée d'extension (29) et qui est mobile entre une position de serrage et une position de libération au moyen d'un dispositif d'actionnement de serrage commun.

6. Orthèse selon l'une des revendications précédentes, **caractérisée en ce que** le dispositif de serrage est constitué par deux anneaux de serrage (30, 31) qui coiffent avec petit jeu le disque porteur de butée de flexion (28) et le disque porteur de butée d'extension (29) sur leur surface périphérique extérieure et qui, dans la position de serrage, empêchent une rotation du disque porteur de butée de flexion (28) et du disque porteur de butée d'extension (29).
